# EUROPEAN PATENT APPLICATION

(11) **EP 3 118 317 A1**
(43) Date of publication of application: **18.01.2017**
(21) Application number: 15759204.9
(22) Date of filing: 29.01.2015
(51) Int. Cl.: C12N 15/12, C12N 15/85, C12N 5/10, A61K 48/00, A61P 9/10, A61P 9/06, A61P 9/04, A61P 9/00

(54) **CODING NUCLEOTIDE SEQUENCE OF MG53 PROTEIN WITH CODON OPTIMIZATION, RECOMBINANT AND USE THEREOF**

(30) Priority: 06.03.2014 CN 201410080068
(71) Applicant: Boya Pharmaceutical Inc., Beijing 100085 (CN)
(72) Inventor: XIAO, Ruiping, Beijing 100085 (CN); CAO, Chunmei, Beijing 100085 (CN); ZHANG, Yan, Beijing 100085 (CN); LV, Fengxiang, Beijing 100085 (CN)
(74) Representative: McNamara, Kathryn
(86) International application number: PCT/CN2015/071808
(87) International publication number: WO 2015/131728

(57) **Abstract**

The present invention relates to a codon-optimized nucleotide sequence encoding MG53, its recombinants and use thereof; the present invention can be applied within the fields of genetic engineering/biomedical technology. Specifically, the present invention relates to a codon-optimized nucleotide sequence encoding MG53, its recombinant vectors harboring the said sequence and host cells. The present invention also relates a method of providing a higher MG53 yield through codon-optimization and its use in biomedical filed.

SEQUENCE LISTING

<110>  BEIJING BOYALIFE PHARMACEUTICALS LTD.

<120>  Codon-optimized nucleotide sequence encoding MG53, its recombinants
and use thereof

<130>  DJ13I0047C

<160>  2

<170>  PatentIn version 3.3

<210>  1
<211>  1434
<212>  DNA
<213>  Homo sapiens

<400>  1
atgtcggctg cgcccggcct cctgcaccag gagctgtcct gcccgctgtg cctgcagctg      60

ttcgacgcgc ccgtgacagc cgagtgcggc cacagtttct gccgcgcctg cctaggccgc     120

gtggccgggg agccggcggc ggatggcacc gttctctgcc cctgctgcca ggcccccacg     180

cggccgcagg cactcagcac caacctgcag ctggcgcgcc tggtggaggg gctggcccag     240

gtgccgcagg gccactgcga ggagcacctg gacccgctga gcatctactg cgagcaggac     300

cgcgcgctgg tgtgcggagt gtgcgcctca ctcggctcgc accgcggtca tcgcctcctg     360

cctgccgccg aggcccacgc acgcctcaag acacagctgc cacagcagaa actgcagctg     420

caggaggcat gcatgcgcaa ggagaagagt gtggctgtgc tggagcatca gctggtggag     480

gtggaggaga cagtgcgtca gttccggggg gccgtggggg agcagctggg caagatgcgg     540

gtgttcctgg ctgcactgga gggctccttg gaccgcgagg cagagcgtgt acggggtgag     600

gcaggggtcg ccttgcgccg ggagctgggg agcctgaact cttacctgga gcagctgcgg     660

cagatggaga aggtcctgga ggaggtggcg gacaagccgc agactgagtt cctcatgaaa     720

tactgcctgg tgaccagcag gctgcagaag atcctggcag agtctccccc acccgcccgt     780

ctggacatcc agctgccaat tatctcagat gacttcaaat tccaggtgtg gaggaagatg     840

ttccgggctc tgatgccagc gctggaggag ctgacctttg acccgagctc tgcgcacccg     900

agcctggtgg tgtcttcctc tggccgccgc gtggagtgct cggagcagaa ggcgccgccg     960

gccggggagg acccgcgcca gttcgacaag gcggtggcgg tggtggcgca ccagcagctc    1020

tccgagggcg agcactactg ggaggtggat gttggcgaca agccgcgctg ggcgctgggc    1080

gtgatcgcgg ccgaggcccc ccgccgcggg cgcctgcacg cggtgccctc gcagggcctg    1140

tggctgctgg ggctgcgcga gggcaagatc ctggaggcac acgtggaggc caaggagccg    1200

cgcgctctgc gcagccccga gaggcggccc acgcgcattg gcctttacct gagcttcggc    1260

gacggcgtcc tctccttcta cgatgccagc gacgccgacg cgctcgtgcc gctttttgcc    1320

ttccacgagc gcctgcccag gcccgtgtac cccttcttcg acgtgtgctg gcacgacaag 1380

ggcaagaatg cccagccgct gctgctcgtg ggtcccgaag gcgccgaggc ctga 1434

<210> 2
<211> 1434
<212> DNA
<213> Artifical Sequence

<220>
<223> Design Based on Codon Usage Bias of Higher Mammals

<400> 2
atgtctgctg ctccaggcct gctgcaccag gaactgtctt gccctctgtg cctgcagctg 60

ttcgacgccc ctgtgaccgc tgagtgcggc cactctttct gcagagcctg cctgggaaga 120

gtggctggcg agcctgctgc tgatggcacc gtgctgtgcc cttgttgcca ggcccctacc 180

agacctcagg ccctgtctac caatctgcag ctggccagac tggtggaagg cctggctcag 240

gtgccacagg gccactgcga agaacacctg gaccccctgt ccatctactg cgagcaggac 300

agagccctcg tgtgcggcgt gtgtgcttct ctgggctctc acagaggcca cagactgctg 360

ccagctgccg aggctcacgc cagactgaaa acccagctgc cccagcagaa gctgcagctg 420

caggaagcct gcatgcggaa agaaaagtcc gtggccgtgc tggaacatca gctggtggaa 480

gtggaagaga cagtgcggca gttcagaggc gctgtgggag agcagctggg caagatgaga 540

gtgttcctgg ccgccctgga aggctccctg gatcgcgagg ctgaaagagt gcggggagaa 600

gctggcgtgg ccctgagaag agagctgggc tccctgaact cctacctgga acagctgaga 660

cagatggaaa aggtgctgga agaggtggcc gacaagcccc agaccgagtt cctgatgaag 720

tactgcctcg tgacctcccg gctgcagaag atcctggccg agtctccacc tcctgcccgg 780

ctggatattc agctgcccat catctccgac gacttcaagt ttcaagtgtg gcggaagatg 840

ttccgggccc tgatgcccgc tctggaagaa ctgaccttcg accccagctc cgcccaccct 900

tctctggtgg tgtcatcctc tggcaggcgg gtggaatgct ccgagcagaa ggctcctcct 960

gctggcgagg accccagaca gttcgataag gccgtggctg tggtggctca ccagcagctg 1020

tctgagggcg agcactactg ggaggtggac gtgggcgata agcccagatg ggctctgggc 1080

gtgatcgccg ctgaggctcc tagaagaggc agactgcacg ccgtgcctag ccagggactg 1140

tggctgctgg gactgagaga gggcaaaatc ctggaagccc acgtggaagc caaagagcct 1200

agagccctgc ggtcccccga gagaaggcct accagaatcg gcctgtacct gtccttcggc 1260

gacggcgtgc tgtccttcta cgacgcctct gatgccgacg ccctggtgcc tctgttcgcc 1320

ttccatgagc ggctgcctcg gcccgtgtac cccttcttcg atgtgtgctg gcacgacaag 1380

ggcaagaacg cccagcctct gctgctcgtg ggacctgaag gtgctgaggc ttga 1434

## Description

### Field of the Invention

The present invention can be applied within the fields of genetic engineering field / bio-medical technology. Specifically, the present invention relates to a codon-optimized nucleotide sequence encoding MG53, its recombinant vectors harboring the said sequences and host cells. The present invention also relates a method of increasing MG53 yield through codon-optimization and its use in biomedical filed.

### Background of the Invention

MG53 (mitsugumin 53, often called as MG53 in abbreviation or TRIM72) is a muscle specific protein of tripartite motif family (TRIM) consisting of three unique motifs as to the RING, the B-BOX, and the coiled-coil domain. The three domains combine together and bind to unnecessary proteins toinduce afterwards degradation through ubiquitination.MG53 is also an essential factor of cell membrane repair mechanism.

In medical therapy aspect, MG53 treatment for cardiac diseases induced by cell apoptosis has been well-recognized and accepted by experts of the field. MG53 has beneficial effects on hearts, which means increased MG53 can protect hearts from injury. However, to our knowledge, MG53 yield cannot be elevated through in-vitro protein expression of wild-type human MG53 gene in eukaryotic cells, therefore MG53 production is low-yield and becomes the bottleneck of MG53's further research and application.

### Description of the Invention

The present invention provides a codon-optimized nucleotide sequence encoding human MG53, SEQ ID:2, referred as the silently-mutated nucleotide sequence of wild-type human MG53 (hereinafter referred to asMG53), which encodes the same human MG53. Wide-type human MG53, SEQ ID NO:1.

MG53 has beneficial effects on hearts, which means increased MG53 can protect hearts from injury. However, numerous studies proved that MG53 yield could not be elevated by means of in-vitro expression of wild-type human MG53 nucleotide sequence, thus the costs for producing MG53 remained high, which prevented this in-vitro expression system from being applied in biomedical industry.

MG53 is a newly found member of the super family of tripartite motif-containing proteins which only expresses in skeletal muscle and myocardium. Early studies demonstrate that MG53 in skeletal muscle and myocardium aids to repair cell membrane injury as well as regulates the transportation of cellular vesicles and the regeneration of skeletal muscle as structural proteins. Besides, MG53 is able to mediate the association of Caveolin-3 and PI3K to activate the reperfusion injury salvage kinase pathway (RISK pathway), thus MG53 plays an important role in protecting hearts during heart ischemia preconditioning. Based on massive experimental data, the inventors of this application provide a codon-optimized nucleotide sequence encoding MG53, referred to as SEQ ID NO:2, and constructs in-vitro expressing systems with eukaryotic vectors and host cells. MG53 yield will rise by some 30% compared with the expressing systems based on wild-type MG53, which makes this nucleotide sequence prominently superior to the wild-type human MG53 nucleotide sequence.

The present invention also provides a method of the codon-optimized MG53 nucleotide sequence in-vitro expression, wherein the method comprises the following steps:
1) Whole-Gene Synthesis of the MG53mut gene on the basis of SEQ ID NO:2;
2) Insert the said MG53 into the eukaryotic expressing vectors through molecular cloning;
3) The recombinant vectors are transfected into the eukaryotic cells, and MG53 are produced by the said cells under the suitable conditions.

In the embodiments, the method of the said silently-mutated MG53 nucleotide sequence (MG53mut) in-vitro expression is as follows:
(1) Whole-Gene Synthesis of the MG53mut on the basis of SEQ ID:2; Taking SEQ ID:2 as a template, and PCR experiments are carried out with DNA polymerase and specially-designed primers, and the PCR product is confirmed by agrose gel electrophoresis;
(2) The PCR product is inserted into the vectors by means of molecular cloning and tranfected into *E coli* competent TOP10: *E.coli* competent TOP10: thaw the competent TOP10, followed by adding PCR product into the cell and incubate on ice, followed by adding LB medium and shaking culture, then spray the cells on LB medium plate with antibiotics, select the single colony for DNA sequencing, the positive colony means successful construction of the MG53mut expression plasmid;
(3) The MG53 protein will be obtained by transfecting the said MG53mut expression plasmids into cells by ScreenFectA.

The present invention also provides a use of MG53mut in preparing the pharmaceutical compositions for treating myocardial injury diseases, wherein the myocardial injury diseases includes myocardial defect, myocardial ischemia injury, myocardial ischemia/reperfusion injury, myocardial infarction, heart failure, cardiac arrhythmia and cardiac rupture.

The use of the MG53 in treating heart injury/myocardial injury, preventing/treating myocardial ischemia and reperfusion injury has been disclosed by the patent CN 200910241451.3.

Over expression of MG53 in cardiomyocytes will protect the cells from injury induced by hypoxia.

### Methods Summary

Reagents and materials: Myc antibodies (Sigma-Aldrich, M4439). Unless indicated otherwise, all chemicals are from Sigma-Aldrich.

Animal models: male rats of same age and source with normal blood pressure are purchased from Vital River Laboratories, Beijing, China. All animal procedures and euthanasia are performed in accordance with protocols approved by the Committee for Animal Research of Peking University, China, and conformed to the Guide for the Care and Use of Laboratory Animals (NIH publication No. 86-23, revised 1985). All mice are maintained in a temperature-controlled barrier facility with a 12-h light/dark cycle and are given free access to food and water in the Center for Experimental Animals at Peking University, Beijing, China (an AAALAC-accredited experimental animal facility).

Plasmids and adenoviral vectors: full-length wild-type MG53 nucleotide sequence is obtained from human muscular tissues by reverse transcription. Silently-mutated MG53 nucleotide sequence is synthesized through gene synthesis. They are inserted into pcDNA4/TO/Myc-His B Expression Vector (Invitrogen) by using BamHI and XhoI restrictive sites.

Cell culture, adenoviral infection and plasmid transfection: C2C12 myoblasts (from Cell Resource Center, IBMS, CAMS/PUMC) are cultured at 37°C under 5% CO2 in Dulbecco's modified Eagle's medium (DMEM) supplemented with 10% fetal bovine serum (FBS, Sigma-Aldrich), 0.11 g/L sodium pyruvate, and 1% penicillin-streptomycin. When C2C12 myoblasts reached 90% confluence, gene transfer is performed by adenoviral infection or plasmid transfection. After gene transfer, cells are cultured in DMEM (2% horse serum) for 4 days to differentiate into myotubes.

Co-immunoprecipitation: Tissues or cells are lysed in lysis buffer A (30mM HEPES at pH7.6,100mM NaCl, 0.5% Nonidet P-40, and protease inhibitors mixture) for 10min at the temperature 4°C, and the lysates are centrifuged at 13,000 r.p.m. for 10 min at 4°C. Co-immunoprecipitation and Western blotting methods have been described previously.

Statistics: using one-way analysis of variance (ANOVA) or repeated-measures ANOVA, when appropriate, with the Bonferroni post-test. All P-values below 0.05 are considered significant. Data are expressed as mean±s.e.m.

### Brief Description of the Drawings

SEQ ID:1 : Wild-type MG53 cDNA sequence (Homo sapiens)
SEQ ID:2 : silently-mutated human MG53 cDNA sequence

Fig.1: Sequence comparison between SEQ ID:1 and SEQ ID:2, MG53 represents wild-type human MG53 cDNA, MG53mut represents silently-mutated MG53 cDNA, and the consensus is the sequence shared by both of them.
Fig.2 wild-type human MG53 and MG53mut recombinant plasmids in agrose gel electrophoresis.

After the bacteria are amplified, the concentration and purity of the wild-type human MG53 and MG53mut recombinant plasmids are confirmed by agrose gel electrophoresis.

Fig.3 Intravenous injection of MG53 will protect rats from myocardial ischemia/reperfusion injury.

Heart pictures show that, compared with BSA group, Intravenous injection of MG53 can significantly reduce the myocardial infarction areas (white areas) induced by myocardial ischemia/reperfusion. The blue areas are non-ischemic areas, and the other areas are ischemic areas. The red parts in the ischemic areas are non-infarcting areas, and the white parts are the infarcting areas.

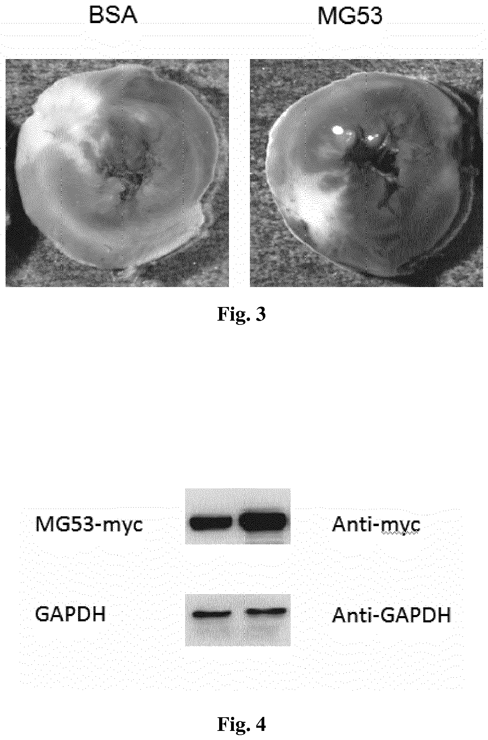

Fig.4 MG53mut reveals a higher MG53 yield than that of wild-type human MG53

Wild-type human MG53 and MG53mut plasmids are transfected into host cells, and 36-48 hours later, MG53 proteinsare harvested and confirmed by western blot.

The top left and bottom left bands in the picture are respectively the MG53 level and GAPDH abundance of wild-type human MG53 plasmids while the top right and bottom right bands are respectively the MG53 level and GAPDH abundance of the MG53mut plasmids.

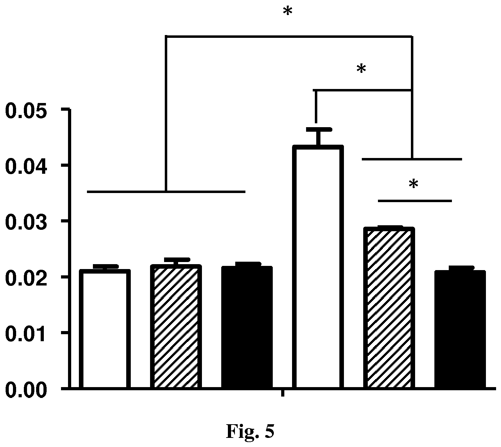

Fig.5 MG53mut and wild-type human MG53 plasmids both have beneficial effects on cardiomyocytes via MG53.

The expression plasmids of MG53mut and wild-type human MG53 are tranfected into H9C2 rat heart cells, then the transfected cells and the control group will go through from the H2O2 -induced oxidative stress injury(simulating the ischemia/reperfusion injury), and then the LDH(cellular injury biomarker) in the medium decreases significantly in the test groups, and the LDH concentration of the MG53mut group is lower than that in wild-type human MG53 group which means both groups are exerting protective effects on the cardiomyocytes against oxidative stress, and the effect of MG53mut group is much stronger.

*means P<0.05, In the bar chart: the blank bar represents the H9C2 control group transfected with empty vectors, the diagonal filling bar represents the wild-type human MG53 group, and the black filling bar represents the MG53mut group. The Y axis means the LDH concentration in the medium (U/ml). The left three bars and the right bars are respectively groups free from and under hypoxia induced by 200um H2O2 for 20 hours.

### Embodiment

***Example 1:*** MG53 cloning, the construction of wild-type human MG53 and MG53mut expression vector.

The total RNAs are extracted from human muscular tissues by TRIZOL.
1) Add 1ml TRIzol into 100mg human muscular tissues, and then homogenize the tissues;
2) Equilibrate the homogenized tissues under room temperature, and add 0.2ml chloroform, and then mix and equilibrate for 3min;
3) Centrifuge the homogenized tissue at 12000g for 15min, and retrieve the supernatant;
4) Add 0.5ml isopropanol into the supernatant, and centrifuge at 12000g for 10min at 4°C, and then discard the supernatant, and wash the RNA with 75% alcohol;
5) Evaporate the RNA and dissolving it again, and then determine its concentration.

### Total RNAs of wild -type MG53 reverse transcription

We use the total RNAsextracts to obtain cDNA. Using the Oligod T as primers, the reverse transcription is conducted by reverse transcriptase to obtain cDNA library.

### The cloning of wild-type MG53 cDNA library

Using the synthesized cDNA library as our template, the PCR is conducted by specific primers to obtain MG53 coding sequence:

### PCR program:

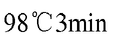

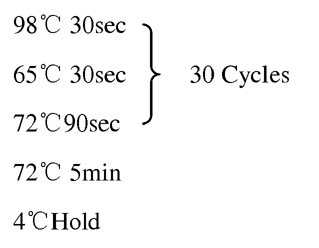

using the program above can we obtain the wild-type MG53 nucleotide sequence(sequence SEQ ID NO:1)

### Primers:

Upperstream: 5'-ata ggatccgccaccatgtcggctgcgcccggcct-3';
Downstream: 5'-atactcgagacggcctcggcgccttcgggacc-3'; carrying the BamHI and XhoI restrictive sites.

PCR product is cut by BamHI and XhoI, and the same procedure is for the empty vector pcDNA4/TO/myc-HisB. The cutting product is retrieved by gel electrophoresis and ligated to the vector by T4 ligase. The sequence is confirmed correct.

The MG53 coding sequence is analyzed and specially designed for silent mutations. Then the MG53mut (SEQ ID NO:2) is obtained by whole-gene synthesis. PCR product is cut by BamHI and XhoI, and the same procedure is for the empty vector pcDNA4/TO/myc-HisB. The cutting product is retrieved by gel electrophoresis and ligated to the vector by T4 ligase. The sequence is confirmed correct.

To obtain the plasmids of MG53 and MG53mut, we use massive plasmids extracting and confirm the quality and concentration of plasmids using agrose gel electrophoresis.

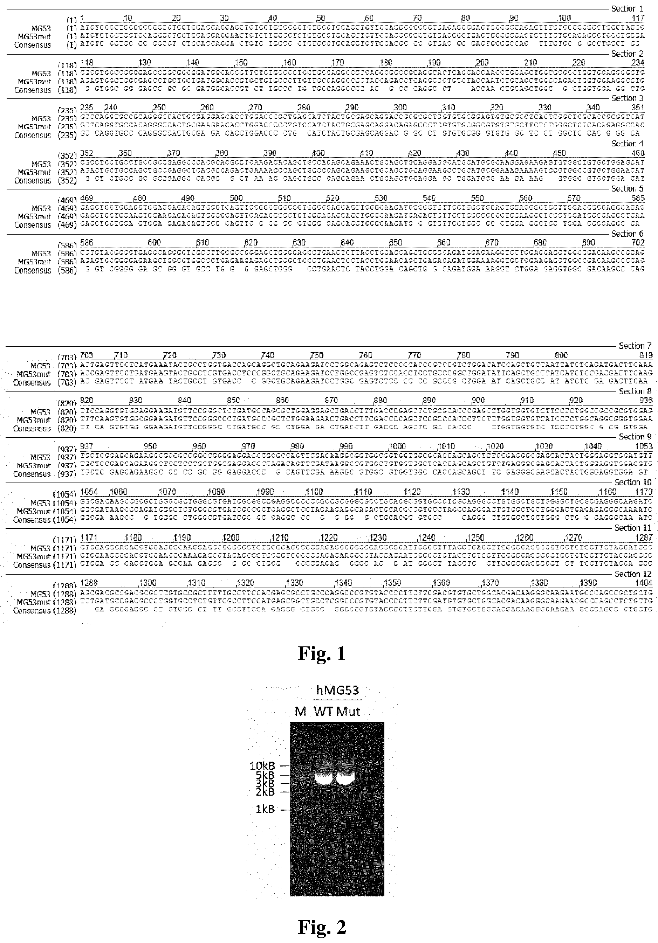

100ml LB medium(1g peptone, 0.5g yeast extract, 1g NaCl, pH 7.5 using NaOH) for TOP10 culture, and centrifuge to obtain the bacteria, adding 5ml lysis buffer(50mM glucose, 25mM Tris-HCl (pH 8.0), 1mM EDTA), vortexing, adding newly-prepared alkaline(200mM NaOH,1%SDS), following equilibrating for 5min, adding 5ml icy-cold 7.5M ammonium acetate(pH7.6), and centrifuge to retrieve the supernatant, then adding 9ml isopropanol and discarding the supernatant, dissolving the sediments by 5ml 2M ammonium acetate(pH7.4), following the centrifuging to obtain the supernatant, and more 5ml isopropanol to settle, at last, the plasmids are extracted by phenol - chloroform and settled by alcohol. The purity and concentration of plasmids are confirmed by agrose gel electrophoresis. See Fig.2.

2. MG53 protects heart from injury.

(1)MG53 and MG53mut gene are expressed in HEK293T cells.

HEK293T cells, cultured in 15cm dish with confluence 80%, are transfected by constructed wild-type human MG53 and silently-mutated MG53mut plasmids with ScreenFect A, Incellar TM). The concrete steps are as follows:
Replace the 293T medium with DMEM, and take one tube for mixing 100uL ScreenFect A and 2000uL dilution buffer together; and another tube for mixing 30ug plasmids with 2000ul dilution buffer; 5min later, mix the two mixtures above together, and the prepared mixture is equilibrated in room temperature for 30min.

Dripping the mixture into the cell culture; 4 hours later, replace the culture with traditional medium (DMEM with 10% FBS). The cells are lyzed after transfection for 36-48 hours and western blot is conducted to determine the protein level by corresponding antibodies. 48 hours later, every dish is lyzed by 1ml Co-IP buffer(30mM HEPES, pH 7.5, 100mM NaCl, 1mM EDTA, 0.5% NP40, protease inhibitor(Roche, 04693132001, one tablet for 50ml buffer)).

### Purify the protein by Myc-tagged affinity method

The concrete steps are as follows:

Wash the anti-myc beads (E6654-1ml, Sigma) 3times by icy-cold PBS, then wash the beads one time by Co-IP buffer. Incubate the beads with lysates for 3 hours at 4°C, and wash the then-beads 5 times with Co-IP buffer and remove the supernatant as much as you can. Then elute the protein by 0.1 M glycine (pH 2.8), the elution buffer is equilibrated by NaCl and Tris-Cl(pH 8.0). The protein is kept in -80°C for use. Small amount of protein is taken to run the SDS-PAGE gel and dyed by Coomassie brilliant blue to determine its purity and concentration compared to BSA marker.

### Example 2: Rat myocardial infarction model establishment and evaluation

Rats (Vital River Laboratories, Beijing, China. SD Rats, body weight: 200g) are anesthetized with pentobarbital sodium (30mg/kg, i.v.) and ventilated by a tracheostomy. A sternotomy is performed on the fourth rib line to open the pericardial membrane and expose the heart. A reversible coronary artery snare occluder was placed around the left anterior descending coronary artery. Myocardial I/R were performed by tightening the snare for 45 minutes and then loosening it for reperfusion. For MG53 treated group, at 5min before ischemia and 1min before reperfusion, MG53 (3mg/KG, i.v.) is injected into the rats. 48 hours later, the rats are anesthetized with pentobarbital sodium (30mg/kg, i.v) and ventilated by a tracheostomy. Open the chests and take off the hearts. The thoracic aorta is ventilated by a tube; and the heart is washed by backward reperfusion of saline. Then snare occluder is placed back to the coronary artery where it was and the artery is dyed with Alcian blue. The dyed areas are the non-ischemic areas, and the undyed areas are the ischemic areas. Then dye the heart section in 1%TTC at 37 °C for 15min, the white parts are infracting areas.
Conclusion: MG53 (i.v.) protects rat heart from ischemia/reperfusion injury. See Fig.3

### Example 3: MG53 and MG53mut expression vector tranfection and expression

HEK293T cells, cultured in 60mm dish with confluence 80%, are transfected by constructed wild-type human MG53 and silently-mutated MG53mut plasmids with ScreenFect A, Incellar TM). The concrete steps are as follows:
Replace the 293T medium with DMEM, then take one tube for mixing 10uL ScreenFect A and 200ul dilution buffer together; and another tube for mixing 3ug plasmids with 200ul dilution buffer; 5min later, mix the two mixtures above together, and the prepared mixture is equilibrated in room temperature for 30min.

Dripping the mixture steadily into the cell culture; 4 hours later, replace the culture with traditional medium (DMEM with 10% FBS). The cells are lyzed after transfection for 36-48 hours and western blot is conducted to determine the protein level by Myc antibodies and GAPDH is to adjust the loading protein amount, see Fig.4. Compared with wild-type MG53 plasmids in same amount of cells, MG53mut plasmids are giving nearly 30% more MG53 yields.

### Example 4: H9C2 heart cells injury induced by hypoxia.

H9C2 heart cells, cultured in 6-well plate, are tranfected by plasmids when reaching confluence 80%: control group: pcDNA4/TO/myc-HisB; human MG53 WT group: wild-type MG53 plasmids; human MG53mut group: silently-mutated MG53 plasmids. The transfection methods are the same. 30 hours later, the culture is replaced with fresh medium supplemented with 200um H2O2. 20 hours later, 40ul medium is sampled and measured for LDH release with the use of a kit (lactate dehydrogenase assay kit, LDH0360, Shanghai Jingyuan Medical Device Company., LTD.) see Fig.5.

## Claims

1. A DNA sequence containing the nucleotide sequence encoding wild-type human MG53 protein, wherein the nucleotide sequence differs from the wild-type human MG53 coding sequences.

2. The DNA sequence of claim 1, wherein the DNA sequence is the nucleotide sequence SED ID NO:2.

3. The DNA sequence of claim 2, wherein the DNA sequence is the nucleotide sequence that one or more than one sites of SED ID NO:2 are silently mutated.

4. A recombinant vector, wherein the recombinant vector contains the nucleotide sequences of any of claims 1-3.

5. The recombinant vector of claim 4, wherein the vector is an animal-expressing vector.

6. The recombinant vector of claim 5, wherein the animal-expressing vector is an adenoviral vector.

7. The recombinant vector of claim 6, wherein the recombinant vector is any vector of pcDNA 4/TO expression vector or pcDNA4 expression vector.

8. The recombinant vector of claim 7, wherein the recombinant vector is pcDNA4/TO/myc-HisB vector.

9. A host cell, wherein the host cell is transfected by any recombinant vector harboring the said DNA sequences of any of claims 4-8.

10. The host cell of claim 9, wherein the host cell is an animal cell.

11. The host cell of claim 10, wherein the host cell is HEK293T cell.

12. A method of increasing wild-type MG53 yield, wherein the method comprises the steps of cultivating the host cells of any of claims 9-11 to obtain wild-type human MG53 under any conditions suitable for protein expression.

13. A use of the DNA sequences of any of claims 1-3 in preparing the pharmaceutical compositions for treating myocardial injury diseases.

14. The use of the DNA sequence of claim 13, wherein the pharmaceutical composition is for myocardial injury diseases including myocardial ischemia injury, myocardial ischemia/reperfusion injury, myocardial infarction, heart failure, cardiac arrhythmia and cardiac rupture.

15. A use of the recombinant vectors of any of claims 4-8 in preparing the pharmaceutical compositions for treating myocardial injury diseases.

16. A use of the wild-type human MG53 obtained by the method of claim 12 in preparing the pharmaceutical compositions for treating myocardial injury diseases.
